Europäisches Patentamt

European Patent Office

Office européen des brevets

(19)

(11) Publication number : **0 615 750 A2**

(12) # EUROPEAN PATENT APPLICATION

(21) Application number : **94301106.4**

(22) Date of filing : **16.02.94**

(51) Int. Cl.⁵ : **A61K 31/00, A61K 31/52, A61K 31/70**

(30) Priority : **17.02.93 GB 9303157**

(43) Date of publication of application :
**21.09.94 Bulletin 94/38**

(84) Designated Contracting States :
**AT BE CH DE DK ES FR GB GR IE IT LI LU MC NL PT SE**

(71) Applicant : **SCOTIA HOLDINGS PLC**
**Efamol House**
**Woodbridge Meadows**
**Guildford Surrey GU1 1BA (GB)**

(72) Inventor : **Horrobin, David Frederick, c/o Scotia Pharma. Ltd.**
**Efamol House,**
**Woodbridge Meadows**
**Guildford GU1 1BA (GB)**
Inventor : **Bond, Peter**
**137 Sheerstock,**
**Haddenham**
**Aylesbury, Bucks HP17 8EY (GB)**

(74) Representative : **Sturt, Clifford Mark et al**
**J. MILLER & CO.**
**34 Bedford Row**
**Holborn**
**London WC1R 4JH (GB)**

(54) **Treatment of a group of related disorders.**

(57)    A method of preparation of a medicament for treatment of one or more of the following conditions when a herpes virus, particularly the herpes simplex virus, is involved in the causation thereof :
Chronic fatigue syndrome
Depression
Irritable bowel syndrome
Fibromyalgia
Headaches
Anxiety
Panic disorder
Narcolepsy
Tourette's disorder
Kleptomania
Bulimia nervosa
Obsessive compulsive disorder
Attention deficit disorder with hyperactivity
Cataplexy
Psychopathic disorder
Gingivitis
Dental caries
wherein a drug effective against said virus is used as an active ingredient of said medicament.

EP 0 615 750 A2

The invention relates to treatment of a group of related disorders which may be caused by Herpes Simplex virus (HSV). It covers the use of therapeutic agents, known to have activity against HSV, in the disorders to be set out below.

One of the inventors has suffered in the past with chronic fatigue syndrome (CFS), associated with loss of concentration and poor memory, with irritable bowel syndrome, persistent headaches, and repeatedly bleeding gums. A course of acyclovir, prescribed for a Herpes virus infection, produced an unexpected and complete remission of the chronic fatigue, the irritable bowel syndrome, the headaches, and the bleeding gums, as well as an improvement in concentration and memory (measured by psychological testing). This raised the possibility that all these conditions may be related to infection by HSV. A search through the literature for associations between these and other disorders was carried out. This search was prompted by the hypothesis that all these conditions might be hitherto unrecognised consequences of persistent infection by HSV. It is, of course, well known that HSV infections are common in the community, but the well recognised lesions consequent on infection and encephalitis are the only diseases currently directly attributed to such infections.

CFS is a condition characterised by disabling fatigue and a variety of other symptoms which occur with varying frequency, low grade fever, myalgias, sleep disorder and impaired cognition being amongst the commonest. It is suggested that it arises in two stages: 1. Depression of the immune system, which can arise from the action of a variety of infectious agents, such as viruses, parasites and bacteria or from stress. 2. Primary infection or reactivation of HSV. HSV could cause the symptoms of CFS by several mechanisms. Firstly, by stimulating the production of interferon in the brain. HSV can spread within the central nervous system, either in the neurons or at a slow speed in the neuroglial cells. In these latter cells it can cause a small area of inflammation, where interferon and other cytokines may be produced. Interferon, given therapeutically, frequently causes "psychiatric" symptoms closely resembling those seen in CFS. Interferon, given prior to surgery for trigeminal nerve decompression, was found to cause a paradoxical increase in HSV reactivation. The "psychiatric" symptoms which can follow therapeutic use of interferon could, therefore be the result of HSV reactivation. Under other conditions, infection of neuroglial cells may result in substantial destruction, perhaps leading to the anaesthesia and paraesthesia seen in some cases of CFS. HSV can also occur in extraneural sites, for example, if the virus is injected into the footpad of the guinea pig, it can still be recovered from this site several months later, despite going into latency in the ganglia. Symptoms could arise from the immune response to HSV in surface tissues such as nasal, oral and gastrointestinal membranes.

Non-invasive brain imaging techniques and neuropsychological testing, indicate a deficit in the temporal lobe in the majority of CFS patients. In human HSV encephalitis, the most common site involved is the temporal lobe. HSV nucleic acid sequences have been detected in the brains of apparently healthy mice, inoculated 24 weeks previously with the virus. They were also found in the brains of elderly patients who had died with psychiatric illness, as well as the majority of normal subjects.

There is much evidence that CFS is related to several other conditions of unknown aetiology. There is considerable overlap between CFS, irritable bowel syndrome (IBS), fibromyalgia and depression. CFS and fibromyalgia are thought by some to be the same disorder, while IBS is listed as a symptom of fibromyalgia. Headache is a common symptom in all these disorders. In a group of patients with new daily persistent headache, all had symptoms other than headache, including fatigue, depression, deficient concentration, paraesthesias, nausea, tinnitus and defective vision, all characteristic of CFS. An almost identical grouping of disorders was arrived at by workers in America, by screening the medical literature for conditions that responded to 3 or more groups of antidepressant treatments. As well as the conditions listed above, they also included narcolepsy, Tourette's disorder, migraine, bulimia, panic disorder, obsessive compulsive disorder, attention deficit disorder with hyperactivity and cataplexy. They called this grouping "affective spectrum disorder". Apart from the evidence of antidepressant response, many phenomenologic and family studies have demonstrated comorbidity between the various conditions in affective spectrum disorder. It was suggested that these disorders shared a common aetiology. HSV is ideally suited to be a causal agent in the conditions that make up affective spectrum disorder.

The experience of one of the present inventors with acyclovir, suggests that HSV may be associated with oral problems. HSV antigen was detected in tissue from around the teeth in the majority of people with clinically healthy gingiva. Acute gingivostomatitis in children is almost invariably due to HSV and it seems possible that reactivation of HSV in adult gingiva could lead to disease. In a study of dental caries, the shedding of HSV was 14 times more frequent than in the controls.

Although acyclovir has been suggested as a treatment for CFS, the only published trial showed it to be ineffective (Straus et al, New England Journal of Medicine 319 No. 26, 1692-1698 (1988). However, we propose that treatment in this case was too short in duration and at such high dosage that toxicity obscured any improvement in well-being. It is also possible that acyclovir may not be the most appropriate drug because it is not lipophilic and as a result may not achieve adequate concentrations in brain and nerve which are the main reservoirs for the virus.

The invention relates to a method of treating at least one of the disorders set out in Table I below by administering to a sufferer on an effective dose/timescale an agent active against a virus of the Herpes class, and in particular against Herpes Simplex virus (HSV).

## Table 1

### The disorders for which we suggest that HSV is a causal agent

Chronic fatigue syndrome

Depression

Irritable bowel syndrome

Fibromyalgia

Headaches

Anxiety

Panic disorder

Narcolepsy

Tourette's disorder

Kleptomania

Bulimia nervosa

Obsessive compulsive disorder

Attention deficit disorder with hyperactivity

Cataplexy

Psychopathic disorder

Gingivitis

Dental caries

The disorders in Table I above are susceptible to treatment with anti-herpes agents.

Suitable anti-herpes agents include acyclovir and gancyclovir. Also suitable for use in the method of the invention are the antiviral agents, active against herpes virus, which are disclosed in EP-A-0 393 920 of the present applicant the disclosure of which application is incorporated herein by reference. These agents include esters, amides or ester/amides of certain unsaturated fatty acids with acyclovir, gancyclovir, azidothymidine (AZT), anhydrothymidine (AHT), dideoxy-didehydro-thymidine (D4T), dideoxycytidine (ddC), dideoxyadenosine (ddA), cytosine arabinoside or adenosine arabinoside. Famcyclovir is an addition to these and all may be

EP 0 615 750 A2

used as the agent itself or as such fatty acid derivatives.

Suitable unsaturated fatty acids include the natural n-7, n-9, n-6 and n-3 series fatty acids, the members of which up to $C_{22}$ are included in Table 2 below:

TABLE 2

| n-7 Diet or bodily synthesis | n-9 Diet or bodily synthesis | n-6 Diet | n-3 Diet |
|---|---|---|---|
| 16:0 ⟶ (palmitic) | 18:0 (stearic) | | |
| **delta 9-desaturase** | | | |
| 16:1 n-7 (palmitoleic | 18:1 n-9 (oleic) | 18:2 n-6 (linoleic) | 18:3 n-3 (alpha-linolenic) |
| **delta 6-desaturase** | | | |
| 16:2 n-7 | 18:2 n-9 | 18:3 n-6 (gamma-linolenic) | 18:4 n-3 |
| 18:2 n-7 | 20:2 n-9 | 20:3 n-6 (dihomo-gamma-linolenic) | 20:4 n-3 |
| **delta 5-desaturase** | | | |
| 18:3 n-7 | 20:3 n-9 | 20:4 n-6 (arachidonic) | 20:5 n-3 |
| 20:3 n-7 | 22:3 n-9 | 22:4 n-6 (adrenic) | 22:5 n-3 |
| **delta 4-desaturase** | | | |
| 20:4 n-7 | 22:4 n-9 | 22:5 n-6 | 22:6 n-3 |

4

These fatty acid derivatives are virucidal and are lipophilic so their penetration through the lipid matrix of the stratum corneum and also their penetration into cells will be efficient. These compounds will be present in the oil phase of oil-in-water or water-in-oil emulsions which simplifies the manufacture and application of such products.

The invention further relates to the use of such anti-herpes agents in the preparation of a medicament for treatment of at least one of the disorders set out in Table 1.

The daily dose according to the invention calculated exclusive of any fatty acid component should be in the range of from 1mg to 200mg per kg per day, preferably from 10mg to 40 mg per kg per day. The treatment should be administered for at least 10 days with the EFA-linked drugs and at least 2 months with those not linked to EFAs. Much longer periods of treatment of up to a year or even indefinitely may be required to prevent recurrence of illness.

The compounds may be administered orally, topically, enterally, parenterally (intravenously, intravascularly, intraperitoneally, intramuscularly or subcutaneously) or by any other appropriate route, all per se known, or using per se known presentations. Thus compounds may be administered as tablets, eg. enteric-coated tablets to prevent destruction in the stomach, hard or soft gelatin capsules, syrups, emulsions, solutions or any other form appropriate for oral or parenteral administration; or any form appropriate for topical administration such as ointments, creams or lotions or as pessaries or other formulations for vaginal or rectal application, or sticks.

## EXAMPLES

1. Tablets of acyclovir, each containing 200 mg or 400 mg of the active drug. To be administered at accumulative daily dose of between 600 and 2000 mg in divided doses evenly during the day over a period of two months or more.
2. Tablets of gancyclovir, famcyclovir, azidothymidine, each containing 100 mg, 200 mg or 400 mg. To be administered at accumulative daily dose of between 400 mg and 2000 mg in divided doses evenly during the day over a period of two months or more.

3-31. The EFA-linked anti-virals of Examples 1-29 of published EPA 0393920, pages 11-16, are formulated as medicaments for the uses set out herein, in one or more of the ways set out at pages 16-18 of that specification, for administration of 10 to 40 mg/kg daily (700 to 2800 mg based on a 70 kg adult, in suitable divided doses) over a period of ten days or more desirably two months or more.

## Claims

1.  A method of preparation of a medicament for treatment of one or more of the following conditions when a herpes virus, particularly the herpes simplex virus, is involved in the causation thereof:

    Chronic fatigue syndrome
    Depression
    Irritable bowel syndrome
    Fibromyalgia
    Headaches
    Anxiety
    Panic disorder
    Narcolepsy
    Tourette's disorder
    Kleptomania
    Bulimia nervosa
    Obsessive compulsive disorder
    Attention deficit disorder with hyperactivity
    Cataplexy
    Psychopathic disorder
    Gingivitis
    Dental caries

    wherein a drug effective against said virus is used as an active ingredient of said medicament.

2.  A method according to claim 1, wherein the drug is selected from acyclovir, gancyclovir, famcyclovir, azidothymidine (AZT), anhydrothymidine (AHT), dideoxy-didehydro-thymidine (D4T), dideoxycytidine (ddC), dideoxyadenosine (ddA), cytosine arabinoside, and adenosine arabinoside.

3. A method according to claim 1 or 2, wherein the drug is in the form of an ester, amide, ester amide or other derivatives of an unsaturated fatty acid of the n-7, n-9, n-6 or n-3 series.

4. A method according to claim 1, 2 or 3, wherein the medicament is for a treatment to be maintained for not less than 10 days with the fatty acid-linked drugs and not less than two months with those not so linked, at a dose of 1 mg to 200 mg/kg body weight daily, desirably 10 mg to 40 mg/kg.